# EUROPEAN PATENT SPECIFICATION

(11) **EP 0 552 586 B1**
(45) Date of publication and mention of the grant of the patent: **20.09.1995**
(21) Application number: 92810872.9
(22) Date of filing: 10.11.1992
(51) Int. Cl.: C07C 49/293, C07C 45/67

(54) **Process for the production of cyclopropylmethylketone**
Verfahren zur Herstellung von Cyclopropylmethylketon
Procédé pour la préparation de cyclopropylméthylkétone

(30) Priority: 19.11.1991 US 794536
(43) Date of publication of application: 28.07.1993
(73) Proprietor: CIBA-GEIGY AG, 4002 Basel (CH)
(72) Inventor: Hunston, Roger, Dr., CH-1870 Blonay (CH); Waditschatka, Rudolf, Dr., CH-5264 Gipf-Oberfrick (CH)

(56) References cited:
- CHEMICAL ABSTRACTS, vol. 86, Columbus, Ohio, US; abstract no. 55055, S. TAKEI et al, "Acylcyclopropanes"; & JP-A-76 105 037
- CHEMICAL ABSTRACTS, vol. 84, Columbus, Ohio, US; abstract no. 105042, S. TAKEI et al, "A novel synthesis of cyclopropyl ketones via decarboxylative ring contractions of alpha-acyl-gamma-butyrolactones catalyzed by halide ions in dipolar aprotic solvents"
- CHEMICAL ABSTRACTS, vol. 84 Columbus, Ohio, US; abstract no. 16711, M. ASAOKA et al, "Alkylation reaction accompanied by dealkoxycarbonylation of beta-keto esters, geminal diesters, and alpha-cyano ester in hexamethylphosphoric triamide (HMPA)"

## Description

The invention relates to a process for the production of cyclopropylmethylketone by heating α-acetyl-γ-butyrolactone in an inert solvent in the presence of a halide, wherein a large excess of the halide is maintained from the start to the end of the reaction.

Cyclopropylmethylketone is used as an intermediate in the manufacture of agrochemicals and pharmaceuticals as described in DE-A-3637788.

The synthesis of cyclopropylketones from acetylbutyrolactones catalysed by an equimolar equivalent of halide ions in dipolar aprotic solvents is known. S.Takei et al. disclose in Tetrahedron Letters (49) pp.4389-92 (1975) a route to cyclopropylketone by reacting α-acetyl-γ-butyrolactone under reflux conditions of 59 hours in dimethylformamide at 160°C in the presence of 1.1 mole equivalents NaBr whereby a yield of only 52% is obtained.

M. Asaoka et al. describe in Chemistry Letters (11) pp.1149-52 (1975) the same reaction of α-acetyl-γ-butyrolactone in hexamethylphosphoric triamide in the presence of NaI or LiCl in a molar ratio of 5:1 (corresponding to 20 mole per cent NaI or LiCl) and a reaction temperature of 170 °C. The cyclopropylmethylketone is removed by distillation during the reaction of 1.5 hours. The yields obtained are 86 % and 42 % respectively. This process is not suitable on an industrial scale due to the low amounts reacted per time unit. One disadvantage of this process is the batch-wise reaction method.

Surprisingly it was found that in the presence of a large excess of halide at any moment of the reaction, by adding continuously the α-acetyl-γ-butyrolactone (hereafter named lactone), and by removing continuously the cyclopropylmethylketone (hereafter named ketone) by distillation, there is a significant increase in the reaction efficiency and turnover, as well as a moderate increase in the yield of ketone. High yields, namely >90%, can be obtained. This reaction can be carried out continuously and on an industrial scale. The amount of halide required per unit of ketone produced is substantially reduced because the halide can be recovered and re-used.

The object of the invention is a process for the production of cyclopropylmethylketone by heating from 160 °C to 220 °C α-acetyl-γ-butyrolactone in the presence of a halide, optionally in an inert solvent, characterized in that i) the halide is selected from the group of LiCl and MXₙ, wherein M is Li^{⊕}, Na^{⊕}, K^{⊕} or quaternary phosphonium when n=1, and Mg^{2⊕} or Ca^{2⊕} when n=2, and X is Br^{⊖} or I^{⊖}, ii) the said acetylbutyrolactone is added throughout the reaction continuously, and simultaneously cyclopropylmethylketone is distilled off continuously, and iii) a large excess of the halide is maintained from the start to the end of the reaction.

A preferred temperature range for the reaction is 160 °C-210 °C and the most preferred range is 170 °C-200 °C.

The reaction can be carried out in an inert solvent or without solvent in the reactant itself. The inert solvent is preferably aprotic. If an alkali or an alkaline earth metal halide is used, the solvent is also preferably dipolar. If a phosphonium halide is used, the solvent can be apolar. It is advantageous for the boiling temperature of the solvent to exceed the reaction temperature by more than 20 °C, preferably by more than 40 °C, for example about 50 °C; in order to avoid co-distillation with the ketone, it is advantageous that the boiling point of the solvent itself exceeds 200 °C.

The solvents used may be, for example: sulfones; sulfoxides; N,N-tetrasubstituted ureas; N-alkylated lactams or N-dialkylated acid amides; ethers; aliphatic, cycloaliphatic or aromatic hydrocarbons, which may be substituted with fluorine, chlorine, or C₁-C₄-alkyl; carboxylic acid esters and lactones; nitriles.

Some specific examples of solvents are:
sulfone: dimethylsulfone, diethylsulfone, tetramethylenesulfone.
sulfoxide: dimethylsulfoxide, diethylsulfoxide.
N,N-tetrasubstituted urea: N-methylethyl-N'-methylethylurea,
N-dimethyl-N'-dipropylurea, tetramethylurea, tetraethylurea,
N,N'-dimethyl-N,N'-1,3-propyleneurea, N,N'-dimethyl-N,N'-ethyleneurea.
N-alkylated lactam: N-methylpyrrolidone, N-ethylpyrrolidone.
N-dialkylated acid amide: N-dimethylformamide, N-diethylformamide,
N-dimethylacetamide.
ether: polyethylglycolether, diethylenglycoldimethylether, diethylenglycoldiethylether.
aliphatic hydrocarbon: nonane, decane
cycloatiphatic hydrocarbon: decahydronaphthalene.
aromatic hydrocarbon: xylene, tetrahydronaphthalene, dichlorobenzene.
carboxylic acid ester: benzoic-methylester.
nitrile: benzonitrile, phenylacetonitrile.

Preferred solvents are N-methylpyrrolidone, tetramethylenesulfone, N,N'-dimethyl-N,N'-1,3-propyleneurea and N,N'-dimethyl-N,N'-ethyleneurea.

The quantity of solvent added is sufficient to essentially dissolve the halide. The concentration of halide in the solvent may be 1-40 volume-%, preferably 2-30 volume-%, more preferably 5-25 volume-%, and most preferably 10-20 volume-%.

Examples of alkali and alkaline earth metal halides are LiCl, LiBr, LiI, NaBr, NaI, KBr, KI, MgBr₂, CaBr₂, MgI₂ and CaI₂. In a preferred embodiment of the invention the reaction is carried out in the presence of alkali metal halides. Preferred alkali metal halides are NaBr and NaI. Iodides are generally preferred over bromides and the most preferred halide is NaI.

Quaternary phosphonium bromides or iodides may be employed which correspond to the formula R₁R₂R₃R₄P^{⊕}X^{⊖} wherein R₁, R₂, R₃ and R₄ represent independently linear or branched C₁-C₂₀, cyclo alkyl of 5-8 ring carbons, phenyl or benzyl, whereby the cyclic residues are unsubstituted or substituted with C₁-C₉-alkyl or C₁-C₉-alkoxy, and X is Br^{⊖} or I^{⊖}.

The cyclic residues are preferably unsubstituted or substituted with C₁-C₆-alkyl or C₁-C₆-alkoxy, for example methyl, ethyl, methoxy or ethoxy.

The groups R₁, R₂, R₃ and R₄ are preferably identical. R₁, R₂, R₃ and R₄ are preferably linear alkyl containing preferably 1-12 carbon atoms and particularly 1-6 carbon atoms, or phenyl or benzyl.

Examples of R₁, R₂, R₃ and R₄ are methyl, ethyl, and the isomers of propyl, butyl, pentyl, hexyl, heptyl, octyl, nonyl, decyl, undecyl, dodecyl, tetradecyl and octadecyl. Preferred alkyl groups are methyl, ethyl, n-propyl, i-propyl, n-butyl, i-butyl, t-butyl.

Examples of cycloalkyls are cyclopentyl and cyclohexyl. Examples of substituted phenyls and benzyls are methylphenyl, methoxyphenyl, methylbenzyl and methoxybenzyl.

Some preferred examples of phosphonium halides are: tetramethyl-, tetraethyl-, tetra-n-propyl-, tetra-n-butyl-, and methyltriphenyl- phosphonium bromide or -iodide.

The reaction can be carried out by placing in the reaction vessel the solvent, optionally the lactone, and all the halide, heating and then adding the lactone.

It is advantageous to dry and deoxygenate the solvent prior to starting the reaction. This can be accomplished as follows: after loading the solvent into the reaction vessel, same is warmed to 60-80 °C and a vacuum applied which is broken with N₂. This process is repeated several times. The solvent is dried by distilling off approximately 1% by volume of the reaction mixture.

The lactone is introduced into the heated reaction vessel to the extent that, at any moment during the reaction, there are present, for example, 1-100, preferably 2-50, and more preferably 3-10 molar equivalents of halide.

The lactone is led into the vessel which has been heated to e.g. 170-220 °C and the ketone begins to distil after saturation of the reaction mixture. The most preferred temperature range is 170-200 °C. It may be advantageous to preheat the lactone.

The lactone is added continuously during the reaction. This continuous process is a preferred embodiment of the reaction.

A particularly effective method of carrying out the continuous process is to use a thin film evaporator (TFE). The reaction mixture of halide, lactone and solvent is fed continuously onto a TFE whose surface is maintained at 180-190 °C. The ketone formed on the evaporator surface is distilled off continuously; the reaction mixture, containing unreacted lactone, is removed from the base of the TFE, the lactone concentration adjusted, and the mixture fed back into the top of the TFE.

A preferred embodiment of the process according to the invention is to distil off the product from the reaction vessel using a semi-continuous fractionation method. A distillation column consisting of a number of plates is connected to the reaction vessel and during the course of the reaction a temperature gradient is established up the column. The product may be collected in an improved quality at the plate number(s) corresponding to the boiling point range of cyclopropylmethylketone. This can be, for example, half way up the column and side products are collected lower down or higher up the column if desired, depending on their respective boiling points.

The reaction is normally carried out at atmospheric pressure, but it may also be carried out at a somewhat reduced pressure or under high pressure when lower boiling temperature solvents are used. The boiling temperature of the solvent is greater than that of the ketone produced.

The advantages of this process are as follows:
a) more efficient production rate of ketone in high yield,
b) much greater turnover of the halide,
c) few side products,
d) more facile and economic production on a commercial scale,
e) the possibility of using no solvent, thus avoiding the necessity of solvent recovery.
f) the halide can be recycled and its consumption is extremely low (less than 1%).

The halide recycling is preferably carried out as follows: the solvent is distilled off and water is added. The organic residue is removed by extraction and the solvent is re-added to the aqueous halide solution. The water is distilled off and the reaction started again.

High turnover of the halide means, for example, that during a reaction process of 100 hours, 1 molar equivalent of halide reacts with approximately 250 molar equivalents of lactone. The product is obtained in a high purity and can be used directly as an intermediate in further reactions. The process is compact, requiring few installations; a vessel volume of 2-4m³ is sufficient to produce several thousand tons of ketone in one year.

The following examples demonstrate the process of the invention.

### EXAMPLES

Examples 1-10 are carried out on a laboratory scale, examples 11, 12 and 13 on a pilot plant scale.

### Example 1:

100 ml dry, deoxygenated solvent and 15 g of NaI are introduced into a 300 ml round-bottomed flask equipped with a thermometer, addition funnel, distillation column, and gas-washer. The mixture is heated to 180 °C and acetylbutyrolactone is introduced at a rate of 50 g/h and CO₂ evolution commences immediately. After a short period, ketone distills at a rate of 30 g/h. After 100 molar equivalents of lactone have been added in relation to NaI, the introduction is stopped and the distillation of ketone allowed to finish. A yield of 775 g (92%) ketone is achieved.

Examples 2-10 are carried out in a similar manner. The following table summarises example results.

| Example No. | Solvent | Halide | Yield CPMK |
|---|---|---|---|
| 1 | NMP | NaI | 92% |
| 2 | NMP | LiBr | 85% |
| 3 | NMP | NaBr | 80% |
| 4 | DMPU | NaI | 92% |
| 5 | DMEU | NaI | 89% |
| 6 | Sulfolane | NaI | 88% |
| 7 | NMP | Bu₄PBr | 85% |
| 8 | Tetralin | Bu₄PBr | 85% |
| 9 | DMPU | LiI | 92% |
| 10 | DMPU | CaI₂ | 80% |
| 11 | NMP | NaI | 90% |
| 12 | DMPU | NaI | 90% |
| Abbreviations CPMK: cyclopropylmethylketone NMP: N-methylpyrrolidone DMPU: N,N'-dimethyl-N,N'-1,3-propyleneurea DMEU: N,N'-dimethyl-N,N'-ethyleneurea Sulfolane: tetramethylenesulfone Tetralin: tetrahydrophthalene Bu: n-butyl | | | |

### Example 11:

25 kg NMP are dried, deoxygenated, and 4.59 kg (30 moles) NaI are added to the vessel which is heated to 180 °C. 386 kg (3000 moles) lactone are fed in constantly over 38 hours which corresponds to a rate of 10 kg/hr or 78 moles/hr. Approximately 227 kg ketone distil off over the 38 hour period.

### Example 12:

25 kg DMPU and 4.59 kg (30 moles) NaI are added to the reaction vessel which is heated to 180 °C. 960 kg (7500 moles) lactone are fed in constantly over 96 hours which corresponds to a rate of 10 kg/hr or 78 moles/hr, the temperature being maintained at 180 °C. Approximately 580 kg ketone distil off over the 96 hour period.

The DMPU is recovered from the reaction mixture by distillation. A vacuum of 30 mbar is applied whilst maintaining a temperature of 160-180 °C in the vessel.

### Recovery of the halide

The NaI is recovered from the residue after solvent distillation by cooling the vessel to 60 °C and adding 25 kg water and 15 kg toluene. The aqueous NaI solution is separated from the organic solution and the NaI recovered after distillation of the water.

### Recycling the halide

The NaI solution is returned into the reactor, and, whilst the DMPU (recovered) is returned into the same reactor, the water is removed by distillation. When the system is anhydrous, the addition of the lactone can be recommenced.

### Example 13

258 kg DMPU and 46 kg (300 moles) NaI are added to the reaction vessel which, after de-oxygenation, is heated to 180 °C. 7670 kg (59,860 moles) lactone are fed in over 96 hours while the temperature is maintained at 180 °C. After a constant temperature gradient is established in the column, approximately 4625 kg of ketone of 98 % quality are drawn off about half way up the column, at the point corresponding to the boiling point of the ketone.

The DMPU is recovered from the reaction mixture by distillation and the NaI is recovered from the residue by extraction.

## Claims

1. A process for the production of cyclopropylmethylketone by heating from 160 °C to 220 °C α-acetyl-γ-butyrolactone in the presence of a halide, optionally in an inert solvent, characterized in that i) the halide is selected from the group of LiCl and MXₙ, wherein M is Li^{⊕}, Na^{⊕}, K^{⊕} or quaternary phosphonium when n=1, and Mg^{2⊕} or Ca^{2⊕} when n=2, and X is Br^{⊖} or I^{⊖}, ii) the said acetylbutyrolactone is added throughout the reaction continuously and simultaneously cyclopropylmethylketone is distilled off continuously, and iii) a large excess of the halide is maintained from the start to the end of the reaction.

2. A process according to claim 1, characterized in that the halide is the bromide or iodide of Na.

3. A process according to claim 1, characterized in that the halide is CaI₂.

4. A process according to claim 1, characterized in that the quaternary phosphonium halide corresponds to the formula R₁R₂R₃R₄P^{⊕}X^{⊖} wherein R₁,R₂,R₃ and R₄ represent independently linear or branched C₁-C₂₀, cyclo alkyl of 5-8 ring carbons, phenyl or benzyl, whereby the cyclic residues are unsubstituted or substituted with C₁-C₉-alkyl or C₁-C₉-alkoxy, and X is Br^{⊖} or I^{⊖}.

5. A process according to claim 4, wherein the cyclic residues are unsubstituted or substituted with C₁-C₆-alkyl or C₁-C₆-alkoxy.

6. A process according to claim 4, wherein R₁,R₂,R₃ and R₄ represent independently C₁-C₁₂-alkyl.

7. A process according to claim 1, characterized in that the halide is essentially dissolved in the inert solvent.

8. A process according to claim 1, characterized in that the halide concentration in the solvent is between 1 and 40 volume-%.

9. A process according to claim 1, characterized in that the halide concentration in the solvent is between 2 and 30 volume-%.

10. A process according to claim 1, characterized in that the halide concentration in the solvent is between 5 and 25 volume-%.

11. A process according to claim 10, characterized in that the halide concentration in the solvent is between 10 and 20 volume-%.

12. A process according to claim 1, characterized in that the halide is present in an excess of 1 to 100 molar equivalents of acetylbutyrolactone at any moment during the reaction.

13. A process according to claim 12 where the excess is from 2 to 50 molar equivalents.

14. A process according to claim 12 where the excess is from 3 to 10 molar equivalents.

15. A process according to claim 1 where the inert solvent is aprotic.

16. A process according to claim 1, characterized in that the boiling temperature of the solvent exceeds the reaction temperature by more than 20 °C.

17. A process according to claim 1 wherein the boiling point of the solvent exceeds the reaction temperature by more than 40 °C.

18. A process according to claim 15 where the solvent is tetramethylenesulfone, dimethylethylurea, dimethylpropylurea, N-methylpyrrolidone, polyethylglycolether or tetrahydronaphthalene.

19. A process according to claim 1, characterized in that the temperature range for the reaction is 160 °C - 210°C.

20. A process according to claim 17, characterized in that the temperature range for the reaction is 170 °C - 200 °C.

21. A process according to claim 1, characterized in that the reaction is carried out at atmospheric pressure.

22. A process according to claim 1, characterized in that the halide is recycled.

23. A process according to claim 22, characterized in that the solvent is distilled off, water added, the organic residue removed by extraction, the solvent re-added to the aqueous halide solution, the water distilled off and the reaction started again.

24. A process according to claim 1, characterized in that the continuous process is carried out in a reaction vessel.

25. A process according to claim 1, characterized in that the continuous process is carried out on a thin film evaporator.

26. A process according to claim 1, characterized in that a distillation column consisting of a number of plates is connected to the reaction vessel, a temperature gradient forms within said column and cyclopropylmethylketone of improved quality is collected at the plate number(s) corresponding to its boiling point range.

## Patentansprüche

1. Verfahren zur Herstellung von Cyclopropylmethylketon durch Erhitzen von α-Acetyl-γ-butyrolacton auf 160°C bis 220°C in Gegenwart eines Halogenids, gegebenenfalls in einem inerten Lösungsmittel, dadurch gekennzeichnet, daß i) das Halogenid ausgewählt ist aus der Gruppe LiCl und MXₙ, worin M Li⁺, Na⁺, K⁺ oder quaternäres Phosphonium darstellt, wenn n=1 ist und Mg²⁺ oder Ca²⁺, wenn n=2 ist, und X Br⁻ oder I⁻ ist, ii) das Acetylbutyrolacton während der gesamten Reaktion kontinuierlich zugegeben wird und Cyclopropylmethylketon gleichzeitig kontinuierlich abdestilliert wird und iii) ein großer Überschuß an Halogenid von Beginn bis zum Ende der Reaktion beibehalten wird.

2. Verfahren nach Anspruch 1, dadurch gekennzeichnet, daß das Halogenid das Bromid oder Jodid von Na ist.

3. Verfahren nach Anspruch 1, dadurch gekennzeichnet, daß das Halogenid CaI₂ ist.

4. Verfahren nach Anspruch 1, dadurch gekennzeichnet, daß das quaternäre Phosphoniumhalogenid der Formel R₁R₂R₃R₄P⁺X⁻ entspricht, worin R₁, R₂, R₃ und R₄ unabhängig voneinander lineares oder verzweigtes C₁-C₂₀-Cycloalkyl mit 5-8 Ringkohlenstoffatomen, Phenyl oder Benzyl wiedergeben, wobei die cyclischen Reste unsubstituiert oder mit C₁-C₉-Alkyl oder C₁-C₉-Alkoxy substituiert sind, und X Br⁻ oder I⁻ ist.

5. Verfahren nach Anspruch 4, wobei die cyclischen Reste unsubstituiert oder mit C₁-C₆-Alkyl oder C₁-C₆-Alkoxy substituiert sind.

6. Verfahren nach Anspruch 4, wobei R₁, R₂, R₃ und R₄ unabhängig voneinander C₁-C₁₂-Alkyl wiedergeben.

7. Verfahren nach Anspruch 1, dadurch gekennzeichnet, daß das Halogenid im wesentlichen in dem inerten Lösungsmittel gelöst ist.

8. Verfahren nach Anspruch 1, dadurch gekennzeichnet, daß die Halogenidkonzentration in dem Lösungsmittel zwischen 1 und 40 Vol.-% ist.

9. Verfahren nach Anspruch 1, dadurch gekennzeichnet, daß die Halogenidkonzentration in dem Lösungsmittel zwischen 2 und 30 Vol.-% ist.

10. Verfahren nach Anspruch 1, dadurch gekennzeichnet, daß die Halogenidkonzentration in dem Lösungsmittel zwischen 5 und 25 Vol.-% ist.

11. Verfahren nach Anspruch 10, dadurch gekennzeichnet, daß die Halogenidkonzentration in dem Lösungsmittel zwischen 10 und 20 Vol.-% ist.

12. Verfahren nach Anspruch 1, dadurch gekennzeichnet, daß das Halogenid in einem Überschuß von 1 bis 100 Moläquivalenten Acetylbutyrolacton zu jedem Zeitpunkt während der Reaktion vorliegt.

13. Verfahren nach Anspruch 12, wobei der Überschuß 2 bis 50 Moläquivalente beträgt.

14. Verfahren nach Anspruch 12, wobei der Überschuß 3 bis 10 Moläquivalente beträgt.

15. Verfahren nach Anspruch 1, wobei das inerte Lösungsmittel aprotisch ist.

16. Verfahren nach Anspruch 1, dadurch gekennzeichnet, daß die Siedetemperatur des Lösungsmittels die Reaktionstemperatur um mehr als 20°C übersteigt.

17. Verfahren nach Anspruch 1, wobei die Siedetemperatur des Lösungsmittels die Reaktionstemperatur um mehr als 40°C übersteigt.

18. Verfahren nach Anspruch 15, wobei das Lösungsmittel Tetramethylensulfon, Dimethylethylharnstoff, Dimethylpropylharnstoff, N-Methylpyrrolidon, Polyethylglycolether oder Tetrahydronaphthalin ist.

19. Verfahren nach Anspruch 1, dadurch gekennzeichnet, daß der Temperaturbereich für die Reaktion 160-210°C ist.

20. Verfahren nach Anspruch 17, dadurch gekennzeichnet, daß der Temperaturbereich für die Reaktion 170-200°C ist.

21. Verfahren nach Anspruch 1, dadurch gekennzeichnet, daß die Reaktion bei Atmosphärendruck ausgeführt wird.

22. Verfahren nach Anspruch 1, dadurch gekennzeichnet, daß das Halogenid zurückgeführt wird.

23. Verfahren nach Anspruch 22, dadurch gekennzeichnet, daß das Lösungsmittel abdestilliert wird, Wasser zugegeben wird, die organischen Rückstände durch Extraktion entfernt werden, das Lösungsmittel erneut zu der wässerigen Halogenidlösung gegeben wird, das Wasser abdestilliert wird und die Reaktion erneut gestartet wird.

24. Verfahren nach Anspruch 1, dadurch gekennzeichnet, daß das kontinuierliche Verfahren in einem Reaktionsgefäß ausgeführt wird.

25. Verfahren nach Anspruch 1, dadurch gekennzeichnet, daß das kontinuierliche Verfahren an einem Dünnfilmverdampfer ausgeführt wird.

26. Verfahren nach Anspruch 1, dadurch gekennzeichnet, daß eine Destillationskolonne, bestehend aus einer Vielzahl von Böden, mit dem Reaktionsgefäß verbunden wird, sich ein Temperaturgradient in der Kolonne bildet und Cyclopropylmethylketon von verbesserter Qualität an der/den Bodenzahl(en), entsprechend seines Siedepunktbereiches, gesammelt wird.

## Revendications

1. Procédé de production de cyclopropylméthylcétone par chauffage entre 160°C et 220°C d'α-acétyl-γ-butyrolactone en présence d'un halogénure, éventuellement dans un solvant inerte, caractérisé en ce que i) l'halogénure est choisi dans le groupe constitué de LiCl et MXₙ dans lequel M représente Li⁺, Na⁺, K⁺ ou un phosphonium quaternaire lorsque n=1, et Mg2⁺ ou Ca2⁺ lorsque n=2, et X représente Br⁻ ou I⁻, ii) on ajoute ladite acétylbutyrolactone pendant la réaction en continu et on récupère simultanément par distillation la cyclopropylméthylcétone en continu, et iii) on maintient un large excès en l'halogénure du début à la fin de la réaction.

2. Procédé selon la revendication 1, caractérisé en ce que l'halogénure est le bromure ou l'iodure de Na.

3. Procédé selon la revendication 1, caractérisé en ce que l'halogénure est CaI₂.

4. Procédé selon la revendication 1, caractérisé en ce que l'halogénure de phosphonium quaternaire correspond à la formule R₁R₂R₃R₄P⁺X⁻, dans laquelle R₁, R₂, R₃ et R₄ représentent indépendamment un groupe en C₁₋₂₀ linéaire ou ramifié, un groupe cycloalkyle à 5 à 8 atomes de carbone par cycle, un groupe phényle ou benzyle, dans lesquels les résidus cycliques sont substitués ou non par des groupes alkyles en C₁₋₉ ou alcoxy en C₁₋₉, et X représente Br⁻ ou I⁻.

5. Procédé selon la revendication 4, dans lequel les résidus cycliques sont substitués ou non par des groupes alkyles en C₁₋₆ ou alcoxy en C₁₋₆.

6. Procédé selon la revendication 4, dans lequel R₁, R₂, R₃ et R₄ représentent indépendamment un groupe alkyle en C₁₋₁₂.

7. Procédé selon la revendication 1, caractérisé en ce que l'halogénure est pratiquement dissous dans le solvant inerte.

8. Procédé selon la revendication 1, caractérisé en ce que la concentration en halogénure dans le solvant est comprise entre 1 et 40% en volume.

9. Procédé selon la revendication 1, caractérisé en ce que la concentration en halogénure dans le solvant est comprise entre 2 et 30% en volume.

10. Procédé selon la revendication 1, caractérisé en ce que la concentration en halogénure dans le solvant est comprise entre 5 et 25% en volume.

11. Procédé selon la revendication 10, caractérisé en ce que la concentration en halogénure dans le solvant est comprise entre 10 et 20% en volume.

12. Procédé selon la revendication 1, caractérisé en ce que l'halogénure est présent dans un excès de 1 à 100 équivalents molaires d'acétylbutyrolactone à tout moment au cours de la réaction.

13. Procédé selon la revendication 12, dans lequel l'excès est compris entre 2 et 50 équivalents molaires.

14. Procédé selon la revendication 12, dans lequel l'excès est compris entre 3 et 10 équivalents molaires.

15. Procédé selon la revendication 1, dans lequel le solvant inerte est aprotique.

16. Procédé selon la revendication 1, caractérisé en ce que la température d'ébullition du solvant dépasse la température de réaction de plus de 20°C.

17. Procédé selon la revendication 1,dans lequel le point d'ébullition du solvant dépasse la température de réaction de plus de 40°C.

18. Procédé selon la revendication 15, dans lequel le solvant est la tétraméthylènesulfone, la diméthyléthylurée, la diméthylpropylurée, la N-méthylpyrrolidone, le polyéthylglycoléther ou le tétrahydronaphtalène.

19. Procédé selon la revendication 1, caractérisé en ce que l'intervalle de température pour la réaction est de 160°C à 210°C.

20. Procédé selon la revendication 17, caractérisé en ce que l'intervalle de température pour la réaction est de 170°C à 200°C.

21. Procédé selon la revendication 1, caractérisé en ce que la réaction est mise en oeuvre à pression atmosphérique.

22. Procédé selon la revendication 1, caractérisé en ce que l'halogénure est recyclé.

23. Procédé selon la revendication 22, caractérisé en ce qu'on élimine le solvant par distillation, on ajoute de l'eau, on élimine le résidu organique par extraction, on réintroduit le solvant dans la solution aqueuse d'halogénure, on élimine l'eau par distillation et on recommence la réaction.

24. Procédé selon la revendication 1, caractérisé en ce que le processus continu est mis en oeuvre dans un récipient de réaction.

25. Procédé selon la revendication 1, caractérisé en ce que le processus continu est mis en oeuvre sur un vaporisateur sur couche mince.

26. Procédé selon la revendication 1, caractérisé en ce qu'une colonne de distillation constituée d'un certain nombre de plateaux est reliée au récipient de réaction, qu'un gradient de température se forme dans la dite colonne et qu'on recueille de la cyclopropylméthylcétone de qualité améliorée au(x) plateau(x) dont les numéros correspondent à sa zone de température d'ébullition.
